# EUROPEAN PATENT APPLICATION

(11) **EP 4 434 452 A1**
(43) Date of publication of application: **25.09.2024**
(21) Application number: 23162774.6
(22) Date of filing: 20.03.2023
(51) Int. Cl.: A61B 5/026, A61B 8/06, A61F 2/01

(54) **PERCUTANEOUS VASCULAR FILTER WITH ULTRASOUND EMBOLUS DETECTION CAPABILITY**

(71) Applicant: Koninklijke Philips N.V., 5656 AG Eindhoven (NL)
(72) Inventor: HINGSTON, John, Eindhoven (NL); ERKAMP, Ramon Quido, Eindhoven (NL); MIEZWA, Megan Ann, 5656AG Eindhoven (NL); CEZO, James David, Eindhoven (NL); DELASSUS, Elodie, Eindhoven (NL); DUIGNAN, Kenneth, Eindhoven (NL)
(74) Representative: Philips Intellectual Property & Standards

(57) **Abstract**

An implantable blood filter system is provided. The system includes an anchor fixedly implantable in a blood vessel of a patient, a filter fixedly attached to the anchor; and an external ultrasound measurement device. The filter includes a plurality of gaps of which size is selected such that blood cells can pass through the gaps, but an embolus in contact with the filter cannot pass through the gaps. The external ultrasound measurement device is configured to detect whether the embolus has been captured by the filter.

## Description

### TECHNICAL FIELD

The subject matter described herein relates to devices, methods, and systems for filtering and detecting emboli in a blood vessel. This implantable blood filter system has particular but not exclusive utility for long-term filtering of the carotid artery.

### BACKGROUND

Stroke is the second leading cause of death globally, and the third leading cause of severe long-term disability. Globally, approximately 14 million stroke cases are estimated to occur annually. There are estimated more than 80 million stroke survivors globally. The United States has 140,000 stroke deaths per year and 7 million stroke survivors, according to the American Stroke Association. According to US Centers for Disease Control and Prevention (CDC), about 800,000 people in the United States suffer from stoke annually, of which 80% of the cases are new.

There are two main types of stroke: ischemic stroke, which is due to the lack of blood flow, and hemorrhagic stroke, which is due to bleeding. Both cause parts of the brain to stop functioning properly. Ischemic stroke represents almost 87% of stroke cases. In the United States, nearly 200,000 of 700,000 ischemic stroke patients (about 30%) receive mechanical thrombectomy treatment. Outside of the United States, 20% of approximately 9.7 million annual cases of ischemic stroke are treated with mechanical thrombectomy.

An embolism is the lodging of an embolus (e.g., any blockage-causing piece of material) inside a blood vessel. An embolism can cause partial or total blockage of blood flow in the affected vessel. Such a blockage (e.g., a vascular occlusion) may affect a part of the body distant from the origin of the embolus. Arterial embolisms are those that follow and, if not dissolved on the way, lodge in a more distal part of the systemic circulation. Arterial embolism can cause occlusion in any part of the body. It is a major cause of infarction (e.g., tissue death from blockage of the blood supply). An embolus lodging in the brain from either the heart or a carotid artery will most likely be the cause of a stroke due to ischemia.

Preventing stroke is one of the most important clinical management goals in the treatment of atrial fibrillation. The pathogenesis of ischemic stroke in atrial fibrillation is most often an embolus emerging from the heart or large arteries and lodging in a cerebral artery. The size of the infarct and extent of brain damage are directly related to the size of the embolus. In Atrial Fibrillation patients, approximately 80% of strokes are total or partial anterior circulation strokes (major strokes) caused by occlusions of the main branches of the carotid arteries. The diameter of these branches is typically greater than 1.5 mm.

Oral anticoagulants are not enough on their own. Warfarin and non-warfarin oral anticoagulants (NOACs) reduce stroke risk by approximately 65%. However, in high-risk patients, the annual stroke risk despite taking oral anticoagulants (OACs) remains high at 2% to 4%. In addition, patients with high stroke risk, plus atrial fibrillation, who are unsuitable for OACs, require other stroke prevention strategies.

The information included in this Background section of the specification, including any references cited herein and any description or discussion thereof, is included for technical reference purposes only and is not to be regarded as subject matter by which the scope of the disclosure is to be bound.

### SUMMARY

Disclosed is an implantable blood filter system having particular, but not exclusive, utility for reducing the occurrence of ischemic stroke. The implantable blood filter system includes a (1) filter that is capable of capturing an embolus while allowing blood cells to pass through, and (2) an external measurement device, probe, or reader that is capable of detecting whether an embolus has been captured by the filter. The filter is implantable (and if necessary, replaceable) via a minimally invasive intravascular procedure. The external measurement device is configured to be usable by the patient in a home setting, with minimal effort or training. If a clot or other embolus is detected within the filter, the patient may be instructed to visit a clinician for appropriate treatment.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes an implantable blood filter system. The implantable blood filter system includes an anchor fixedly implantable in a blood vessel of a patient; a filter fixedly attached to the anchor; and an external ultrasound measurement device. The filter includes a plurality of gaps having a gap size, where the gap size is selected such that: blood cells can pass through the plurality of gaps; and an embolus in contact with the filter cannot pass through the plurality of gaps and the embolus is captured by the filter. The external ultrasound measurement device is configured to detect whether the embolus has been captured by the filter. Other embodiments of this aspect include corresponding computer systems, apparatus, and computer programs recorded on one or more computer storage devices, each configured to perform the actions of the methods.

Implementations may include one or more of the following features. In some embodiments, to detect whether the embolus has been captured by the filter, the external ultrasound measurement device is configured to detect an ultrasound spectrum of the embolus. In some embodiments, an ultrasound signature of the filter is configured to change in response to the filter being captured by the embolus, and where, to detect whether the embolus has been captured by the filter, the external ultrasound measurement device is configured to detect the changed ultrasound signature. In some embodiments, the filter includes ultrasound markers. In some embodiments, the filter includes a plurality of filter stages. In some embodiments, the external ultrasound measurement device includes a handheld device. In some embodiments, the external ultrasound measurement device includes a wearable device. Implementations of the described techniques may include hardware, a method or process, or computer software on a computer-accessible medium. In some of the embodiments the ultrasound signature may be associated with a change in ultrasound Doppler signal.

One general aspect includes a permanently implantable blood filter. The permanently implantable blood filter includes a stent fixedly implantable in a blood vessel of a patient; and a filter fixedly attached to the stent, where the filter includes a coil or mesh, where the coil or mesh includes a plurality of gaps having a gap size, where the gap size is selected such that: blood cells can pass through the plurality of gaps and an embolus in contact with the filter cannot pass through the plurality of gaps and the embolus is captured by the filter.

Implementations may include one or more of the following features. In some embodiments, the stent and coil or mesh each have an acoustic impedance between 1.2 and 2.3 Mrayls (MegaRayls). In some embodiments, at least one of the stent or filter further includes ultrasound markers with an acoustic impedance of between 5 and 50 Mrayls, or between 0 and 0.6 Mrayls.

One general aspect includes a measurement device. The measurement device includes an array of ultrasound transducers; a user interface configured to provide visual, auditory, or haptic feedback to a user; a processor in communication with the user interface and the array of ultrasound transducers; and a memory operatively coupled to the processor and including instructions that direct the processor to: with the array of ultrasound transducers, detect whether an embolus is present in a filter device implanted within the user; and with the user interface, indicate to the user whether the embolus is present in the filter device. Other embodiments of this aspect include corresponding computer systems, apparatus, and computer programs recorded on one or more computer storage devices, each configured to perform the actions of the methods.

Implementations may include one or more of the following features. In some embodiments, the instructions further direct the processor to: with the user interface, indicate whether the measurement device is properly placed or oriented to detect the embolus in the filter device. In some embodiments, the instructions further direct the processor to: if the processor does not detect the filter device, provide, with the user interface, a first indication; or if the processor detects the filter device, provide, with the user interface, a second indication. In some embodiments, the instructions further direct the processor to: if the processor detects that the embolus is not present in the filter device, provide, with the user interface, a third indication; or if the processor detects that the embolus is present in the filter device, provide, with the user interface, a fourth indication. In some embodiments, at least one of the first indication, the second indication, the third indication, or the fourth indication is a visual, auditory, or haptic indication. In some embodiments, the measurement device is a handheld device. In some embodiments, the measurement device is a wearable device. Implementations of the described techniques may include hardware, a method or process, or computer software on a computer-accessible medium.

One general aspect includes a method. The method includes implanting a filter device in a vessel of a patient; with a handheld or wearable measurement device, detecting whether an embolus is present in the filter device; and with a user interface, alerting the user whether the embolus is detected to be present in the filter device. Other embodiments of this aspect include corresponding computer systems, apparatus, and computer programs recorded on one or more computer storage devices, each configured to perform the actions of the methods.

Implementations may include one or more of the following features. In some embodiments, the method further includes: if the embolus is detected to be present in the filter device, introducing anticoagulant medications into the patient. In some embodiments, the method further includes: if the embolus is detected to be present in the filter device, removing the filter device. Implementations of the described techniques may include hardware, a method or process, or computer software on a computer-accessible medium.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the implantable blood filter system, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
**Figure 1** is a schematic, diagrammatic, perspective view of at least a portion of an example implantable blood filter system, in accordance with at least one embodiment of the present disclosure.
**Figure 2** is a schematic, diagrammatic, cross-sectional view of at least a portion of an example temporary implantable blood filter deployment system, in accordance with aspects of the present disclosure.
**Figure 3** is a perspective image of a temporary carotid artery filter, in accordance with aspects of the present disclosure.
**Figure 4** is a schematic, diagrammatic, side cross-sectional view of at least a portion of an example implantable blood filter system, in accordance with at least one embodiment of the present disclosure.
**Figure 5** is a schematic, diagrammatic, end cross-sectional view of at least a portion of an example implantable blood filter system, in accordance with at least one embodiment of the present disclosure.
**Figure 6** is a schematic, diagrammatic, end cross-sectional view of at least a portion of an example implantable blood filter system, in accordance with at least one embodiment of the present disclosure.
**Figure 7** is a schematic, diagrammatic, perspective view of at least a portion of an example implantable blood filter system, in accordance with at least one embodiment of the present disclosure.
**Figure 8** is a schematic, diagrammatic, perspective view of at least a portion of an example implantable blood filter system, in accordance with at least one embodiment of the present disclosure.
**Figure 9** is a diagrammatic, schematic view of a handheld medical scanning device, according to aspects of the present disclosure.
**Figure 10** is a diagrammatic, perspective view of a user applying an external measurement device to the site of the implant to perform a self-test, in accordance with at least one embodiment of the present disclosure.
**Figure 11** is a diagrammatic, perspective view of a user wearing a wearable external measurement device, ultrasound probe, or reader over the site of the implant to perform a self-test, in accordance with at least one embodiment of the present disclosure.
**Figure 12** is a flow diagram showing the steps of an example implantable permanent blood filter placement method, in accordance with at least one embodiment of the present disclosure.
**Figure 13** is a schematic diagram of a processor circuit, according to embodiments of the present disclosure.

### DETAILED DESCRIPTION

In accordance with at least one embodiment of the present disclosure, a implantable blood filter system is provided that includes a permanent percutaneous carotid artery filter with embolus/clot detection capabilities. The carotid filter device is designed to be permanently implanted in the carotid artery of a patient (e.g., implanted for weeks, months, years, decades, or the lifespan of the patient), and the filter is designed such that it is straightforward to detect, using external ultrasound, when an embolus/clot has been captured.

In the current state of the art, deployable carotid artery filters (embolic protection devices) are normally placed before a carotid stent procedure to capture any embolus/clot, as a result from stenting procedure. However, the embolic protection devices are temporary, and need to be removed at the end of the procedure.

There is a significant need for other stroke prevention therapies. The present disclosure outlines a novel solution with a permanent percutaneous carotid artery filter designed to be easily tested using external ultrasound, to ascertain if it has captured an embolus or not. The filter has specific features in its design to facilitate the detection of a captured embolus by a low skilled operator. For example, the design is such that the patient can perform the ultrasound scan at home, with minimal training, to check if the filter has captured an embolus. If the filter has captured an embolus, or is suspected of having captured an embolus, the patient can then go to the local hospital to remove the embolus, e.g., with anticoagulants. However, if that fails to dissolve the embolus, the filter can be removed during a minimally invasive procedure and replaced with a new filter.

The permanent carotid filter is designed such that when the filter captures an embolus, the ultrasound signature of the filter changes in a significant and easily detectable way. The filter comes with a 'reader', that the patient can use at home to regularly check for captured embolus. For example, on a regular basis (e.g., once per week, once per day, etc., depending on risk level) the patient does a simple test at home to check if the filter captured an embolus.

The filter device may be anchored in place using a number of different state of the art options. For example, the filter may be anchored using radial force. In a manner similar to traditional stents, the device may exert an outward radial force on the blood vessel that keeps the filter in place. To achieve this, the stent/filter could be of a number of different options, including but not limited to a Nitinol braided stent, a Nitinol laser cut stent, a stainless alloy laser cut stent, a stainless steel braided stent, a polymer braided stent, or a polymer laser cut stent. Instead or in addition, to prevent migration, the filter may be anchored by one or more sutures, or by any other method known in the art.

The filter part of the device has the ability to filter the blood flowing through the carotid artery, leveraging current state of art, and to have a detectably different ultrasound signature between its normal state (e.g., no embolus captured) and non-normal state (e.g., an embolus has been captured in the device). The filter may be or include a polymer net or mesh, a coil or spiral, an elongated pattern, or other filter type known in the art, and may include multi-stage filters as shown below. The filter and anchor may for example be designed to have a very low ultrasound signature. When an embolus is captured in the filter, the embolus itself will typically have a high ultrasound signature that is readily detectable by the external probe. In other embodiments, high-ultrasound-signature markers may be incorporated into the filter such that when an embolus is captured, changes in the filter shape result in changes to the ultrasound signature.

The external test device may for example be a standard, off-the-shelf external ultrasound probe system, with software specific to detecting an embolus in the implanted filter and delivering a simple yes/no answer that is readily understandable by the patient. In other embodiments, the external test device may be a wearable device such as a neck wrap.

The present disclosure provides not only a permanently implantable blood filter, but also devices and methods for a low skilled user, for example the individual patient, to detect a captured embolus, with minimal effort and training. If an embolus has been detected, the patient can seek prompt medical attention to resolve the embolus. Thus, the implantable blood filter system may significantly reduce the incidence and/or severity of ischemic stroke (or other embolus-related ischemic injury) in the patients for whom it is deployed, and may therefore improve patient outcomes. This may be accomplished at relatively low cost, as the blood filter is implantable (and if necessary, replaceable) via a minimally invasive intravascular procedure, and the testing can be performed in a home setting, without the need for clinician involvement.

The present application is related to U.S. Application No. 63/440,877, filed on January 24, 2023, hereby incorporated by reference in its entirety as though fully set forth herein. These descriptions are provided for exemplary purposes only, and should not be considered to limit the scope of the implantable blood filter system. Certain features may be added, removed, or modified without departing from the spirit of the claimed subject matter.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

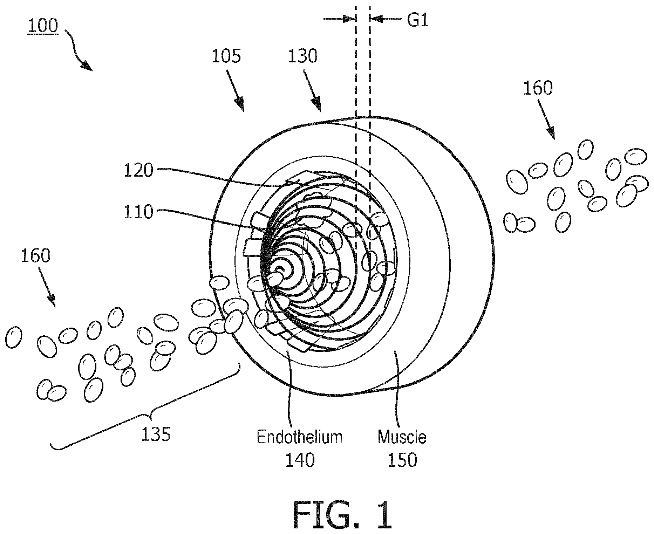

**Figure 1** is a schematic, diagrammatic, perspective view of at least a portion of an example implantable blood filter system 100, in accordance with at least one embodiment of the present disclosure. The implantable blood filter system 100 includes an implant 105 comprising a filter 110 and an anchor 120, which are implantable in a blood vessel 130. The blood vessel 130 includes an endothelium or vessel wall 140 surrounded by muscle tissue 150. The blood vessel 130 carries blood 135, including blood cells 160.

In some embodiments, the filter 110 and anchor 120 are designed to have a very low ultrasound signature. When an embolus is captured in the filter, the embolus itself will generally have a high ultrasound signature that is readily detectable by the external probe, such that a strong echo being returned from the vicinity of the filter is indicative of a captured embolism.

An implant 105 with low ultrasound signature could be fabricated from plastic materials that are currently used in plastic stents. Compared to metal materials, plastic materials have an acoustic impedance that is closer to the acoustic impedance of blood (e.g., approximately 1.66 MegaRayls or Mrayls). The lower impedance mismatch creates weaker reflections and therefore a lower ultrasound signature. In addition, the ultrasound signature can be further lowered by using materials that are significantly thinner in the ultrasound imaging beam direction that the ultrasound wavelength (e.g., a wavelength of ~300 micron at 5MHz). Avoiding flat surfaces parallel to the transducer face also minimizes the ultrasound signature. An example of a plastic with acoustic impedance close to that of blood is polyethylene, with an impedance of approximately 1.73 Mrayls, as compared with (for example) stainless steel, with an acoustic impedance of 45.7 Mrayls, or polymethyl methacrylate, with an acoustic impedance of 3.26 Mrayls. In general, plastics with an acoustic impedance between 1.2 and 2.3 Mrayls may be considered to have a small ultrasound signature when implanted in the bloodstream, as this is symmetrical reflection range around the 1.66 Mrayls acoustic impedance of blood, which has a reflection coefficient of about 2.6% for a surface boundary between the materials. This is less than the 3.28% reflection coefficient from a skin/fat boundary layer, and leaves sufficient acoustic energy to pass through and image below the material boundary. Other ranges, both larger and smaller, may be used instead or in addition. The anchor 120 and filter 110 may both be made of plastic, such that the entire implant 105 is made of plastic with a low ultrasound signature. When an embolus is captured by the filter, this can therefore be readily detected by the external measurement device, as an embolus generally returns a strong ultrasound echo, whereas blood returns a weaker echo.

The filter includes gaps of a width G1, which is large enough to allow blood cells 160 to pass through, but small enough to prevent the passage of an embolus large enough to cause an ischemic stroke, or other ischemic injury. Depending on the implementation, the frame or anchor 120 may be or include at least one of a Nitinol braided stent, a Nitinol laser cut stent, a stainless alloy laser cut stent, a polymer braided stent, a polymer laser cut stent, or may be or include one or more sutures.

Before continuing, it should be noted that the examples described above are provided for purposes of illustration, and are not intended to be limiting. Other devices and/or device configurations may be utilized to carry out the operations described herein.

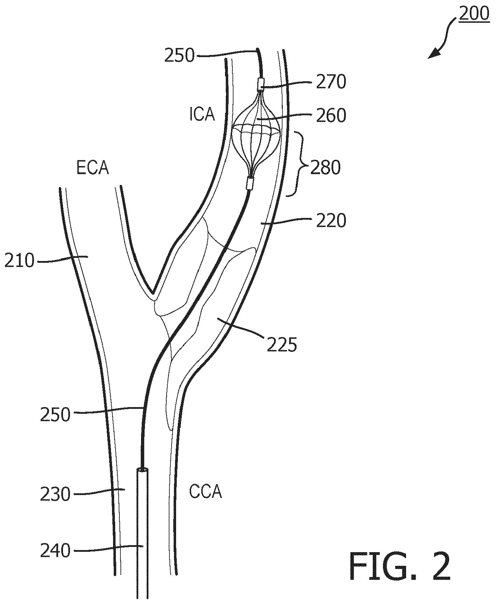

**Figure 2** is a schematic, diagrammatic, cross-sectional view of at least a portion of an example temporary implantable blood filter deployment system 200, in accordance with aspects of the present disclosure. Visible are the external carotid artery, (ECA) 210, the internal carotid artery (ICA) 220 which includes a constriction 225, the common carotid artery (CCA) 230, a catheter 240, and a guidewire 250. The temporary implantable blood filter deployment system 200 includes a temporary carotid artery filter 260 that is placed downstream (e.g., cranial) of a constriction 225 before a stenting or ablation procedure. In the example shown in Figure 2, the temporary filter 260 is incorporated directly into the guidewire 250, and can be opened and closed by a deployment mechanism 280 that, under the control of a clinician, is translated proximally and distally relative to an anchor 270.

The temporary filter 260 is placed only during the ablation or stent placement procedure, to capture material that may be dislodged during the procedure. In most cases, the temporary filter 260 must be removed after the stent is placed or the ablation is complete.

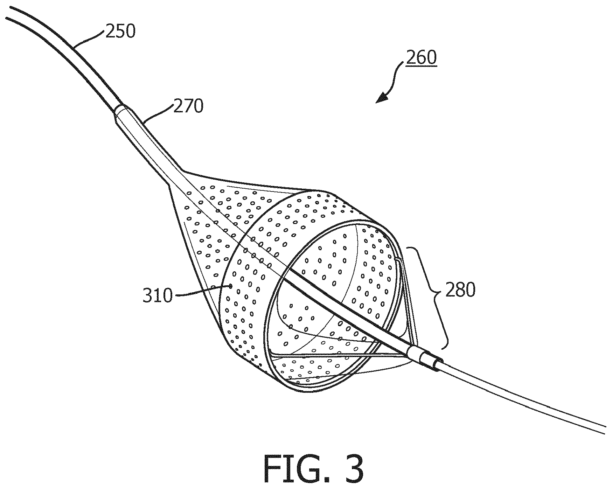

**Figure 3** is a perspective image of a temporary carotid artery filter 260, in accordance with aspects of the present disclosure. Visible are the guidewire 250, anchor 270, temporary filter 260, and deployment mechanism 280. The temporary filter 260 may for example be made of a polymer mesh that includes holes 310. The holes 310 are large enough to allow blood cells to pass, but small enough to capture an embolus that is capable of causing ischemic stroke.

The design of the temporary filter 260 is such that it is anchored by the catheter. It is not designed to be left in place, as it has no mechanism for anchoring to the wall of a blood vessel.

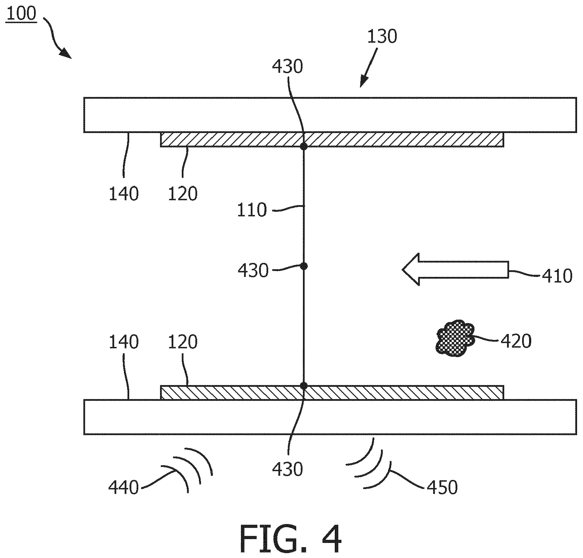

**Figure 4** is a schematic, diagrammatic, side cross-sectional view of at least a portion of an example implantable blood filter system 100, in accordance with at least one embodiment of the present disclosure. Visible are the filter 110 and frame or anchor 120. The filter 110 may be co-formed with, or fixedly attached to, the frame or anchor 120. The frame or anchor 120 exerts radially outward pressure on the vessel wall 140, thus preventing the filter 110 from migrating within the blood vessel 130. In the example shown in Figure 4, an embolus 420, moving in the blood flow direction 410, can be captured by the filter 110.

Also visible are ultrasound waves 440 (e.g., emitted by an external ultrasound probe or "reader"), and ultrasound reflections or echoes 450 reflected from the filter 110 and frame 120. In some embodiments, detection of the embolus 420 (e.g., by an external ultrasound sensor) relies on the ultrasound signature of the embolus itself, which may return a significantly stronger ultrasound echo 450 than the blood vessel 130, frame or anchor 120, and filter 110 collectively do. However, in other embodiments the implant includes ultrasound-detectable features or ultrasound markers 430 that are arranged in a particular pattern (e.g., a straight line, as shown), whose shape - and therefore ultrasound signature - changes when an embolus 420 is captured. For example, the tip or center of the filter may be made from, or otherwise incorporate, a piece of high ultrasound signature material (an `ultrasound marker') 430. When the filter captures an embolus, the high-ultrasound-signature material physically moves, and this change can be readily detected by an ultrasound probe as a change in intensity of the ultrasound echoes 450. In still other embodiments, the frame 120 and/or filter 110 may be made of high-ultrasound-signature materials such as metals, and an ultrasound probe may detect the presence of an embolus 420 either by the change in shape (and thus ultrasound signature) of the filter 110, or by the change in ultrasound signature due to the embolus 420 obscuring some portions of the filter 110 and/or frame 120. In each case, however, the shape and materials of the filter 110 and frame 120 are selected such that a detectable change in ultrasound signature (e.g., in the intensity of ultrasound echoes 450) occurs when an embolus 420 is captured by the filter 110.

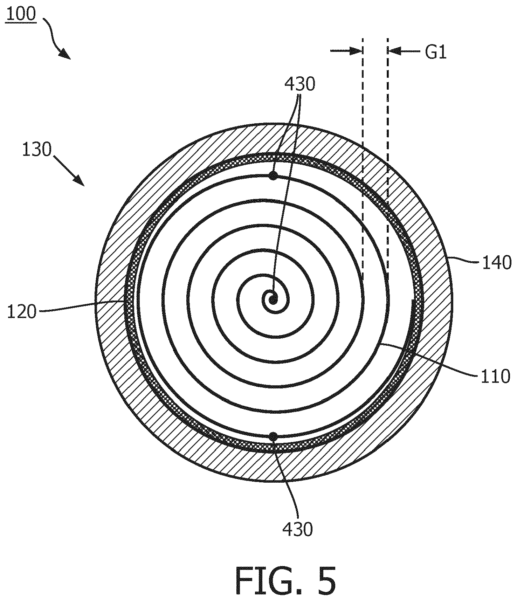

**Figure 5** is a schematic, diagrammatic, end cross-sectional view of at least a portion of an example implantable blood filter system 100, in accordance with at least one embodiment of the present disclosure. Visible are the vessel wall 140 of the blood vessel 130, along with the frame or anchor 120 and the filter 110. In the example shown in Figure 5, the filter 110 is or includes a spiral of a material such as plastic or metal. In other embodiments, the filter 110 may be or include a mesh, grid, perforated sheet, or other related structure that includes gaps of a width G1, which is large enough to allow blood cells 160 to pass through, but small enough to prevent the passage of an embolus large enough to cause an ischemic stroke, or other ischemic injury.

In some embodiments, the filter 110 includes ultrasound markers 430 arranged in a particular pattern (e.g., a straight line, as shown), such that the ultrasound signature of the filter 110 changes when an embolus is captured.

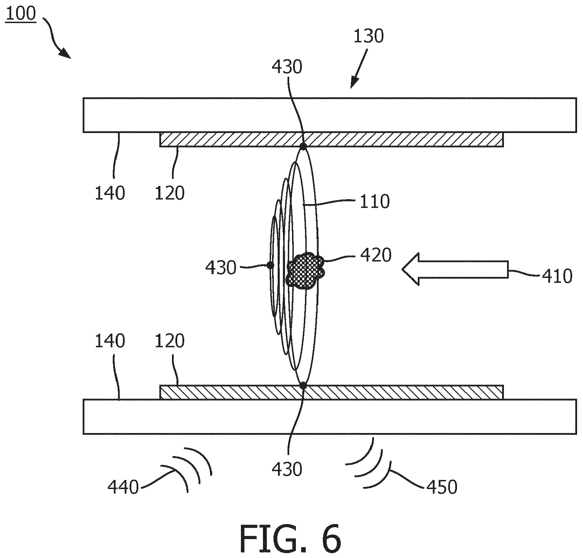

**Figure 6** is a schematic, diagrammatic, end cross-sectional view of at least a portion of an example implantable blood filter system 100, in accordance with at least one embodiment of the present disclosure. Visible are the vessel wall 140 of the blood vessel 130, the frame or anchor 120, the filter 110, ultrasound markers 430, ultrasound waves 440, and ultrasound echoes 450. The arrangement of Figure 6 is similar to that shown in Figures 4 and 5, except that the embolus 420, moving in the blood flow direction 410, has been captured by the filter 110, resulting in a deformation of the filter 110 such that the ultrasound markers 430 are no longer in their original arrangement (e.g., a predefined geometry such as straight line, coplanar, etc). The resulting change in the ultrasound signature of the filter 110 (e.g., a change in the intensity of the ultrasound echoes 450) can be detected by an external ultrasound probe or reader.

The ultrasound markers 430 may for example be made of a material that has very different acoustic impedance that the surrounding media (blood and other implant components), thereby enhancing acoustic reflections from its surfaces. The acoustic impedance of blood is typically around 1.66 Mrayls. Suitable ultrasound marker materials can have either very high acoustic impedance (gold with 23.2 Mrayls, platinum with 36 Mrayls), or near zero acoustic impedance by entrapment of air bubbles in for example a polymer (air with 0.0004 Mrayls). In an example, the ultrasound markers have an acoustic impedance between 5 and 50 Mrayls, or between 0 and 0.6 Mrayls, close to the reflection that Z=5 would give when in blood. These ranges allow at least about 22% reflection coefficient at surface between materials, thus making the boundary easier to detect. Other ranges, both larger and smaller, may be used instead or in addition. Performance of the ultrasound markers can be further enhanced by ensuring sufficient thickness in the direction of the ultrasound beam and surface orientations favorable to generating strong ultrasound echoes 450.

In other embodiments, ultrasound markers 430 are not present, and the captured embolus 420 is detected by the ultrasound signature of the embolus 420 itself, or by the ultrasound signal of a high-acoustic-impedance filter 110 and/or a high-acoustic-impedance frame or anchor 120 being partially blocked by the embolus 420.

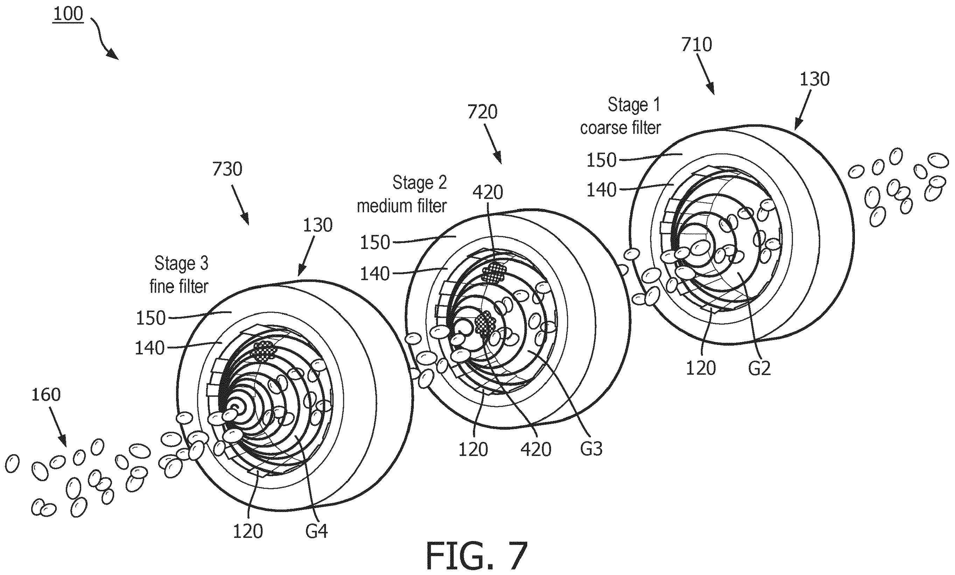

**Figure 7** is a schematic, diagrammatic, perspective view of at least a portion of an example implantable blood filter system 100, in accordance with at least one embodiment of the present disclosure. In the example shown in Figure 7, the implantable blood filter system 100 includes three frames or anchors 120 implanted within the blood vessel 130 through an outward radial force exerted on the vessel wall 140 and muscle tissue 150. In the example shown in Figure 7, the implantable blood filter system 100 includes three filters: a first filter or coarse filter 710, a second filter or medium filter 720, and a third filter or fine filter 730. In an example, the first filter or coarse filter 710 includes gaps G2 that are small enough to trap a large embolus 420, but large enough to let blood cells 160 and smaller emboli 420 pass through. Similarly, the second filter or medium filter includes gaps G3 that are small enough to trap emboli 420 of moderate or typical size, but large enough to let blood cells 160 and small emboli 420 pass through, while the third filter or fine filter includes gaps G4 that are small enough to trap small emboli 420, but large enough to let blood cells 160 pass through.

The use of three filters is merely an illustrative example that does not limit the scope of the present disclosure. Other numbers of filters may be used instead or in addition, including two, four, five, or more filters, or continuous filters. The advantage of a multi-stage filter is that it can reduce the resistance of blood flow past the filter, e.g. by decreasing the pressure differential before and after the filter. With multistage filters or continuous filters, the filter mechanism can be spread out over a length of the blood vessel. For example, the stage 1 filter could be most radially (outside edge) focused, while stage 2 is focused on the mid-section of the blood vessel, and stage 3 is concentrated at or near the center section.

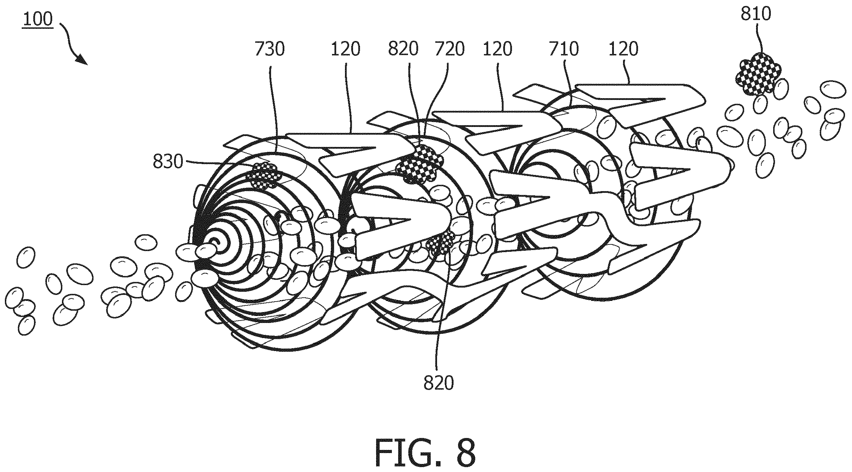

**Figure 8** is a schematic, diagrammatic, perspective view of at least a portion of an example implantable blood filter system 100, in accordance with at least one embodiment of the present disclosure. The view of Figure 8 is similar to Figure 7, with the vessel wall 140 and muscle tissue 150 of the blood vessel deleted for clarity. Although each filter or filter stage 710, 720, 730 is shown with its own anchor 120 in Figure 7, it is noted that in some embodiments, two or more filters or filter stages may be held in place by a single anchor 120, or with at least one anchors shared by two or more filters that also serve as connection between them, like illustrated in Figure 8.

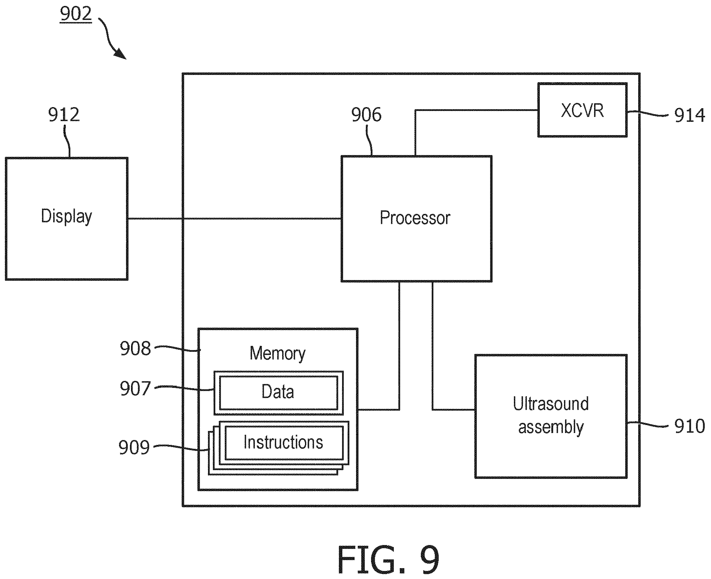

**Figure 9** is a diagrammatic, schematic view of a handheld medical scanning device 902, according to aspects of the present disclosure. The handheld medical scanning device 902 includes an ultrasound assembly 910. The ultrasound assembly may include a single ultrasound transducer, a 1D array of transducers, or a 2D array of transducers. The ultrasound assembly emits ultrasound waves and receives ultrasound reflection or echoes. Depending on the implementation, either or both of the ultrasound waves or the ultrasound echoes may be beamformed or otherwise processed.

The handheld medical scanning device 902 may include a memory 908 with data 907 and a plurality of instructions 909 stored therein, an ultrasound assembly 910, a display 912, and a radiofrequency transceiver 914 each in communication with a processor 906.

The memory 908 may comprise a non-volatile memory and/or a volatile memory. The data 907 may comprise any type of data including medical data such as: imaging data, e.g., imaging data files, assessment results, diagnoses, measurements, treatment plans, medication schedules, test results, appointment schedules, progress reports, a patient history, etc. The plurality of instructions 909 may comprise instructions which, when executed by the processor 906, cause the processor to perform one or more of the techniques described herein. For example, the plurality of instructions 909 may comprise one or more of heuristic algorithms, machine learning algorithms, device positioning algorithms, algorithms for processing imaging data, algorithms for segregating relevant imaging data from irrelevant imaging data, etc.

The processor 906 may operate the ultrasound assembly 910, which may be miniaturized, and which may comprise an ultrasound transducer array in some instances, to obtain imaging data and may receive such imaging data from the ultrasound assembly 910. The processor 906 may generate images based on the received imaging data and may output such images, e.g., ultrasound images, which may comprise 3D ultrasound images, to the display 912. The processor 906 may also receive user input via the display 912, or may communicate information to the user via the display 912. For example, the display 912 may indicate to the user whether the handheld medical scanning device 902 is correctly positioned or correctly aligned with respect to the implantable filter, or may indicate to the user whether or not a captured embolus has been detected in the implantable filter.

The processor 906 may operate the radiofrequency transceiver 914 to communicate with a remote medical processing system, e.g., a hospital record system or other hospital system. The radiofrequency transceiver 914 may be configured to communicate over an Institute of Electrical and Electronics Engineers (IEEE) 802.11 (WiFi) link, a Bluetooth link, a Zigbee link, an ultra-wideband (UWB) link, or over any combination thereof. In some cases, the handheld medical scanning device 902 may comprise multiple radiofrequency transceivers 914, and different radiofrequency transceivers 914 may be configured to communicate over different links. In that regard, different radiofrequency transceivers 914 may be configured to communicate over different frequencies, different time slots, etc.

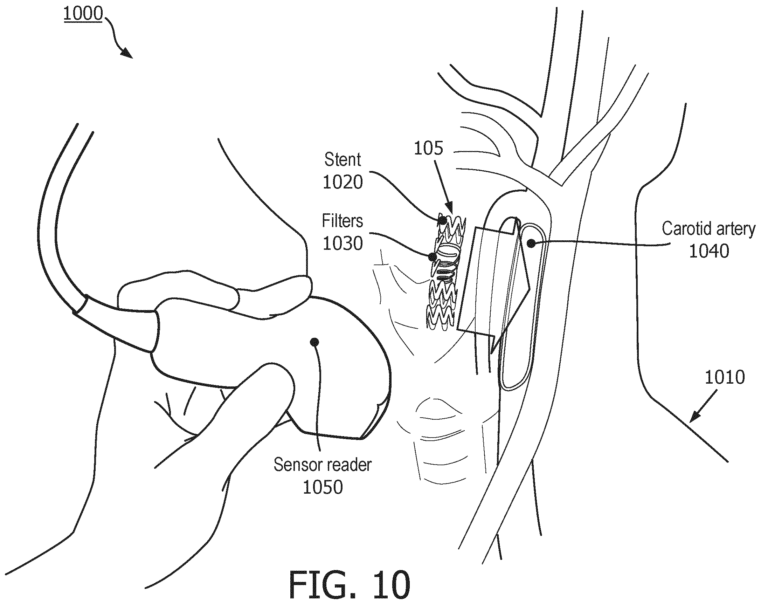

**Figure 10** is a diagrammatic, perspective view of a user 1010 applying an external measurement device, ultrasound probe, or reader 1050 to the site of the implant 105 in the carotid artery 1040 to perform a self ultrasound test, in accordance with at least one embodiment of the present disclosure. The implant 105 may include a frame, anchor, or stent 1020 and one or more filters 1030. The stent and/or filter(s) are disposed in the carotid artery, as the arrow shows their location.

A key feature of the present disclosure is that when an embolus has been captured, it easily shows up on an ultrasound test. To achieve this, both the implant 105 and the external measurement device 1050 can be designed or customized with the specific intent of performing this detection in a home setting, with minimal training. Thus, although the external measurement device, ultrasound probe, or reader 1050 may be an off-the-shelf instrument or may include off-the-shelf components, it may be customized (through any combination of software, firmware, or hardware) such that it primarily or exclusively detects the presence or absence of a captured thrombus within the filters 1030 of the implant 105. For example, the external measurement device 1050 may provide a first indication (e.g., an auditory tone) indicating that it does not currently detect the implant 105, and a second indication (e.g., a different audio tone) when the position and orientation of the external measurement device 1050 are such that the implant 105 can be detected. Then, based on the ultrasound signal, the external measurement device 1050 can provide a third indication (e.g., a green light) that a captured embolus has not been detected within the implant 105, or a fourth indication (e.g., a red light) that a captured embolus has been detected within the implant 105. The indications may for example be through a user interface providing any combination of visual, auditory, and/or haptic feedback to the user. In some instances, the user interface may be in the external measurement device 1050 itself. In other instances, the user interface may be at least partially within a user device such as a mobile phone, tablet, notebook, laptop, or desktop computer. Thus, the external measurement device 1050 provides a fast and easy test to ascertain if any filter 1030 of the implant 105 has captured any emboli.

In an example, once per x days (for example every day, every 3 days, every 7 days, etc., depending on the patient's perceived risk level), the patient places the external measurement device 1050 over their neck to check if the filter has captured an embolus.

An algorithm in the sensor device could analyze the collected ultrasound data to assess if an embolus is present and alert the patient. Alternatively, the collected ultrasound data could be automatically transmitted to a healthcare provider for further analysis. The external measurement device 1050 may include a transceiver that connects to the patient's personal smartphone, and an app that patients install on their personal smartphone for analyzing the collected ultrasound data.

In an example, the patient is trained to know where to place the external measurement device 1050 over the implant in the carotid artery. The patient applies gel to the probe end of the external measurement device 1050. The patient places the probe end on the spot they've been trained to place it. In some cases, the spot may be marked with a tattoo or other marking. The patient then performs a 'sweep', by changing the angle of the external measurement device 1050. The external measurement device 1050 then detects whether an embolus has been captured in the implant 105. The external measurement device 1050 alerts the patient to seek medical attention if a clot or other embolus in the filter has been detected.

The external measurement device 1050 may employ one or more methods to detect a captured embolus. Simply detecting the intensity of ultrasound echoes received from the device is one such method. Instead or in addition, Doppler ultrasound imaging can be used to detect flow patterns of blood. In the presence of an occlusion in the filter, the pattern of the blood flow changes in detectable ways. At the location of the embolus, there may be little or no flowing blood. There may, however, be a clot, thrombus, or other embolus that could be vibrating or moving around and thus creating its own Doppler signal. If the filter design minimized thrombus vibration, associated Doppler signals from it could be minimized and also velocity ranges could be markedly different from surrounding blood and therefore allow differentiation between the blood and the embolus. The presence of an embolus also locally narrows the lumen of the vessel, and therefore increases the blood flow velocity in the remaining lumen around the embolus. Thus, higher doppler velocity values can indicate presence of a thrombus. An embolus could also cause turbulence and therefore alter the distribution of blood velocities within the vessel. By detecting these blood flow pattern changes, the external measurement device 1050 can detect the presence of an embolus in the implant 105.

Harmonic imaging may also be employed. For example, ultrasound markers on the filter can be designed such that they are particularly efficient at being detected using harmonic ultrasound. Harmonic ultrasound signals are generated due to nonlinearities in the acoustic behavior of the medium through which the ultrasound beam is traveling. For example, gas bubbles are known to generate strong harmonic signals, as they vibrate due to pressure variations from the acoustic imaging beam (which is why ultrasound contrast agents may incorporate small microbubbles). In some embodiments, parts of the filter 1030 are configured such that they have similar behavior as ultrasound contrast agents. For example, some of the fibers in the filter mesh could be made very compliant and thin, and formed as hollow, gas-filled tubes.

It is noted that the external measurement device 1050 may employ one or more sensing techniques that include detection of sound waves at any frequency. Examples include, but are not limited to, low-frequency ultrasound, high-frequency ultrasound, photoacoustic sensing, or otherwise, with or without an imaging capability, that is capable of discerning whether an embolus has been captured by the filter. The ultrasound sensor technology could be based on piezoelectric ceramics such as PZT, capacitive micromachined ultrasound transducers (CMUT), polymer-based piezoelectric materials such as PVDF, or otherwise.

If the external measurement device 1050 indicates that the filter did capture an embolus, the patient goes to hospital for supervision, and may for example be given anti-thrombolytics to dissolve the embolus. If anti-thrombolytics are unsuccessful, the implant 105 device can be removed and replaced with a new implant 105, in a minimally invasive procedure.

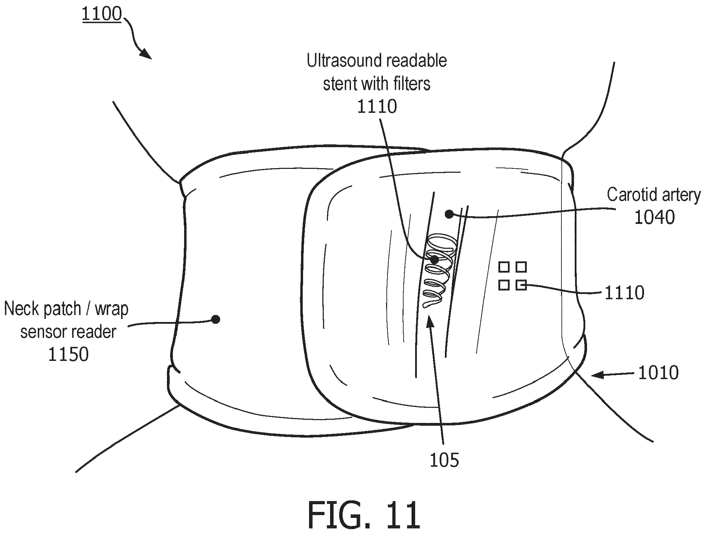

**Figure 11** is a diagrammatic, perspective view of a user 1010 wearing a wearable external measurement device, ultrasound probe, or reader 1150 over the site of the implant 105 in the carotid artery 1040 to perform a self ultrasound test, in accordance with at least one embodiment of the present disclosure. The wearable external measurement device 1150 may for example take the form of a neck wrap that incorporates an array of ultrasound transducers 1110. In some embodiments, the wearable external measurement device 1150 may include external markings to help the user align the transducers 1110 with the implant 105. In other embodiments, the wearable external measurement device 1150 includes ultrasound transducers 1110 over a large area, and activates the transducers 1110 that are closest to the implant 105 to perform the detection of the implant 105 and/or embolus.

In an example, the imaging transducers 1110 integrated into the neck wrap include a method for performing a sweep of the ultrasound imaging plane along the carotid. This may be a mechanical mechanism to move the transducer assembly, either manually by the patient or through a motorized actuation. Alternatively, the transducer assembly may be a stationary 2D ultrasound array that allows for electronically sweeping the imaging plane through a volume of interest. In an example, the user would apply ultrasound gel to their neck before wrapping the wearable external measurement device 1150 around it. Operation of the wearable external measurement device 1150 may otherwise be similar to operation of the external measurement device 1050 of Figure 10.

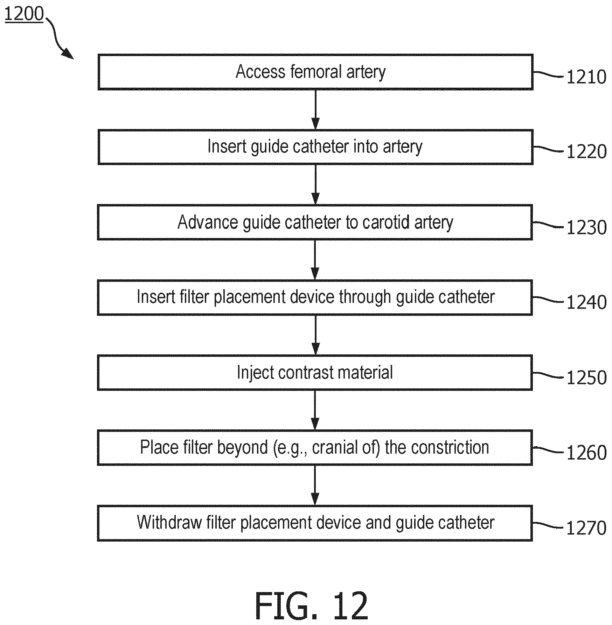

**Figure 12** is a flow diagram showing the steps of an example implantable permanent blood filter placement method 1200, in accordance with at least one embodiment of the present disclosure. For ease of adoption, the method 1200 may be similar to those currently used for implanting a temporary filter in the carotid artery.

In step 1210, the clinician surgically accesses the femoral artery.

In step 1220, the clinician places a sheath or guide catheter into the femoral artery.

In step 1230 the clinician advances the guide catheter to the carotid artery.

In step 1240, the clinician inserts a filter placement device (e.g., a filter placement guidewire) through the guide catheter, and advances it into the carotid artery. In some cases, A catheter with a balloon tip may then be threaded through the guide catheter to a narrowing in the carotid artery (e.g., under X-ray guidance).

In step 1250, the clinician injects contrast material into the carotid artery through the guide catheter and/or balloon catheter, in order to locate a constriction in the carotid artery. In many cases, the most suitable landing site for a permanent filter is downstream of (e.g. cranial of) the constriction. However, even in cases where no constriction is present, the contrast agent may enable the clinician to select a suitable landing site for the filter.

In step 1260, the filter is placed in the artery. The filter, also called an embolic protection device, may for example be inserted cranial of a construction to catch any debris that may break off from the narrowed area of artery during a stenting, balloon angioplasty, or ablation procedure.

In step 1270, the clinician withdraws the filter placement device, guide catheter, and any other intravascular instruments from the patient, and closes access to the femoral artery. The method is now complete.

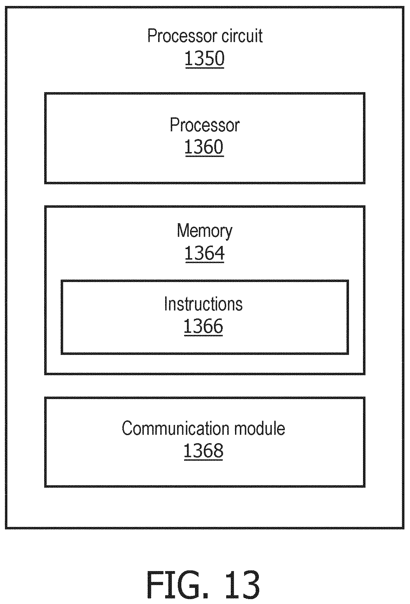

**Figure 13** is a schematic diagram of a processor circuit 1350, according to embodiments of the present disclosure. The processor circuit 1350 may be implemented in the external measurement device 1050 or 1150, or other devices or workstations (e.g., third-party workstations, network routers, etc.), or on a cloud processor or other remote processing unit, as necessary to implement the method. As shown, the processor circuit 1350 may include a processor 1360, a memory 1364, and a communication module 1368. These elements may be in direct or indirect communication with each other, for example via one or more buses.

The processor 1360 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1360 may also comprise another hardware device, a firmware device, or any combination thereof configured to perform the operations described herein. The processor 1360 may also be implemented as a combination of computing devices, e.g., a combination of a DSP and a microprocessor, a plurality of microprocessors, one or more microprocessors in conjunction with a DSP core, or any other such configuration.

The memory 1364 may include a cache memory (e.g., a cache memory of the processor 1360), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1364 includes a non-transitory computer-readable medium. The memory 1364 may store instructions 1366. The instructions 1366 may include instructions that, when executed by the processor 1360, cause the processor 1360 to perform the operations described herein. Instructions 1366 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1368 can include any electronic circuitry and/or logic circuitry to facilitate direct or indirect communication of data between the processor circuit 1350, and other processors or devices. In that regard, the communication module 1368 can be an input/output (I/O) device. In some instances, the communication module 1368 facilitates direct or indirect communication between various elements of the processor circuit 1350 and/or the external measurement device 1050 or 1150. The communication module 1368 may communicate within the processor circuit 1350 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the external measurement device) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles), 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

As will be readily appreciated by those having ordinary skill in the art after becoming familiar with the teachings herein, the implantable blood filter system advantageously provides not only a filter designed explicitly for permanent or long-term implantation in a blood vessel, but also devices and methods for detecting an embolus that has been captured by the filter, in a home setting, with minimal training and effort.

A number of variations are possible on the examples and embodiments described above. For example, the implantable blood filter system can be employed in veins as well as arteries, and in peripheral vasculature as well as cardiac or pulmonary vasculature (e.g., the vena cava), or the vasculature of other body organs including but not limited to the liver, kidneys, and brain. The implantable blood filter system may be readily detectable through physical inspection and/or inspection of promotional materials. The word "embolus" should be interpreted as including any blood clot, gas bubble, thrombus, dislodged tissue (e.g., fat, plaque, scar tissue, webbing, etc.), or other blockage-causing material large enough to present a risk of ischemic injury to the patient. The term "ultrasound" should be interpreted as including any sensing method that includes detection of ultrasonic waves emitted or reflected by a boundary, that is capable of discerning whether an embolus has been captured by the filter, including but not limited to photoacoustic measurement, B-mode imaging, Doppler signal processing, extraction of harmonic signals within the ultrasound signal, and utilizing various sensor materials (PZT, CMUT PVDF), with or without an imaging capability.

The implantable blood filter system can be used for stroke prevention, stenting procedures, and any procedure where a filter (e.g., a temporary filter) is currently placed in the venous or arterial system. The technology described herein may be sized, shaped, or composed differently than shown herein, without departing from the spirit of the present disclosure.

The logical operations making up the embodiments of the technology described herein are referred to variously as operations, steps, objects, elements, components, or modules. It should be understood that these may occur, or be performed or arranged, in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the implantable blood filter system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the implantable blood filter system as defined in the claims. Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

## Claims

1. An implantable blood filter system, comprising:
an anchor fixedly implantable in a blood vessel of a patient;
a filter fixedly attached to the anchor; and
an external ultrasound measurement device,
wherein the filter comprises a plurality of gaps having a gap size,
wherein the gap size is selected such that:
blood cells can pass through the plurality of gaps; and
an embolus in contact with the filter cannot pass through the plurality of gaps and the embolus is captured by the filter; and
wherein the external ultrasound measurement device is configured to detect whether the embolus has been captured by the filter.

2. The system of claim 1, wherein, to detect whether the embolus has been captured by the filter, the external ultrasound measurement device is configured to detect an ultrasound spectrum of the embolus.

3. The system of claim 1,
wherein an ultrasound signature of the filter is configured to change in response to the filter being captured by the embolus, and
wherein, to detect whether the embolus has been captured by the filter, the external ultrasound measurement device is configured to detect the changed ultrasound signature.

4. The system of claim 3, wherein the filter comprises ultrasound markers.

5. The system of any of the preceding claims, wherein the filter comprises a plurality of filter stages.

6. The system of any of the preceding claims, wherein the external ultrasound measurement device comprises a handheld device or a wearable device.

7. A permanently implantable blood filter, comprising:
a stent fixedly implantable in a blood vessel of a patient; and
a filter fixedly attached to the stent,
wherein the filter comprises a coil or mesh,
wherein the coil or mesh comprises a plurality of gaps having a gap size,
wherein the gap size is selected such that:
blood cells can pass through the plurality of gaps and
an embolus in contact with the filter cannot pass through the plurality of gaps and the embolus is captured by the filter.

8. The permanently implantable blood filter of claim 7, wherein the stent and coil or mesh each have an acoustic impedance between 1.2 and 2.3 Mrayls.

9. The permanently implantable blood filter of claim 7 or 8, wherein at least one of the stent or filter further comprises ultrasound markers with an acoustic impedance of between 5 and 50 Mrayls, or between 0 and 0.6 Mrayls.

10. A measurement device, comprising:
an array of ultrasound transducers;
a user interface configured to provide visual, auditory, or haptic feedback to a user;
a processor in communication with the user interface and the array of ultrasound transducers; and
a memory operatively coupled to the processor and comprising instructions that direct the processor to:
with the array of ultrasound transducers, detect whether an embolus is present in a filter device implanted within the user; and
with the user interface, indicate to the user whether the embolus is present in the filter device.

11. The measurement device of claim 10, wherein the instructions further direct the processor to:
with the user interface, indicate whether the measurement device is properly placed or oriented to detect the embolus in the filter device.

12. The measurement device of claim 10, wherein the instructions further direct the processor to:
if the processor does not detect the filter device, provide, with the user interface, a first indication; or
if the processor detects the filter device, provide, with the user interface, a second indication.

13. The measurement device of claim 12, wherein the instructions further direct the processor to:
if the processor detects that the embolus is not present in the filter device, provide, with the user interface, a third indication; or
if the processor detects that the embolus is present in the filter device, provide, with the user interface, a fourth indication.

14. The measurement device of claim 10, wherein the measurement device is a handheld device or a wearable device.

15. The system of any of the claims 1 to 6, wherein the permanently implantable blood filter is according to any of the claims 7 to 9 and wherein the external ultrasound measurement device is according to any of the claims 10 to 14.
